# EUROPÄISCHE PATENTSCHRIFT

(11) **EP 0 518 151 B1**
(45) Veröffentlichungstag und Bekanntmachung des Hinweises auf die Patenterteilung: **06.09.1995**
(21) Anmeldenummer: 92109191.4
(22) Anmeldetag: 01.06.1992
(51) Int. Cl.: C07C 205/37, C07C 205/38, C07C 201/12

(54) **Verfahren zur Herstellung von Nitrophenyl-alkylethern**
Process for the preparation of nitrophenyl-alkylethers
Procédé pour la préparation d'éthers de nitrophényle et d'alkyle

(30) Priorität: 14.06.1991 DE 4119664
(43) Veröffentlichungstag der Anmeldung: 16.12.1992
(73) Patentinhaber: BAYER AG, 51368 Leverkusen (DE)
(72) Erfinder: Beitzke, Bernhard, Dr., W-5064 Rösrath (DE); Weitz, Robert, Dr., W-5060 Bergisch Gladbach 2 (DE)

(56) Entgegenhaltungen:
- FR-A- 1 532 235
- WORLD PATENTS INDEX LATEST Week 8442, Derwent Publications Ltd., London, GB; AN 84-261555 & RO-A-84 290
- WORLD PATENTS INDEX LATEST Week 9041, Derwent Publications Ltd., London, GB; AN 90-310197 & JP-A-2 221 239
- 90---310197

## Beschreibung

Die Erfindung betrifft ein Verfahren zur Herstellung von Nitrophenyl-alkylethern aus Nitrophenolen und Alkylchloriden in Gegenwart von Chlorwasserstoff bindenden Verbindungen, das dadurch gekennzeichnet ist, daß in Wasser als Reaktionsmedium gearbeitet wird und ferner Nitrophenol in Wasser vorgelegt wird und das Alkylchlorid und die Chlorwasserstoff bindende Verbindung simultan zugesetzt werden.

Nitrophenyl-alkylether sind wichtige organische Zwischenprodukte, beispielsweise zur Herstellung von Farbstoffen.

Aus Beilstein, 4. Auflage, Band VI, S. 412 (1923) ist die Herstellung von 2-Nitro-4-methyl-phenyl-methylether aus trockenem 2-Nitro-p-cresol in Form des Silbersalzes und Methyliodid, welches mit Ether verdünnt ist, bekannt.

Aus Houben-Weyl, 4. Auflage, Band VI/3 (1965), S. 54ff. ist die Alkylierung von Phenolaten mit Alkylhalogeniden in wäßrigem Medium bekannt. Sobald jedoch die Phenolate eine Nitrogruppe tragen, wird diese Umsetzung in einem organischen Lösungsmittel, wie Cyclopentanon oder Xylol (loc. cit., S. 56) vorgenommen. Auch in Fierz-David, Grundlegende Operationen der Farbenchemie, 8. Auflage (1952), S. 143, wird Methanol bzw. Ethanol als das Reaktionsmedium angegeben.

Des weiteren ist die Benutzung eines anderen Alkylierungsmittels, nämlich Dimethylsulfat im Zusammenwirken mit Kaliumcarbonat in Xylol als Lösungsmittel bekannt (Beilstein, 4. Auflage, Band VI EI, S. 206).

Aus RO 84290 (zitiert nach Derwent Publications AN 84-261 555) ist bekannt, zur Herstellung von Alkyl-p-nitrophenyl-ethern das p-Nitrophenol oder p-Nitrophenol-Na-salz und Wasser einem Alkylchlorid, das einen Phasentransferkatalysator enthält, zuzusetzen und die Reaktanden gemeinsam auf 50-100°C zu erhitzen.

Aus JP 2/221 239 (zitiert nach Derwent Publications AN 90-310 197) ist bekannt, phenolische Verbindungen, die auch durch Nitro substituiert sein können, in wäßrigem Medium mit Methylbromid umzusetzen.

Den Verfahren des Standes der Technik haften verschiedene Nachteile an. So ist die Vorlage des gesamten Nitrophenols in Form des Phenolates ein Sicherheitsrisiko und führt ferner zu Problemen beim Rühren und zu einem schlechten Umsatz wegen der Schwerlöslichkeit des Nitrophenolats. Legt man alle Reaktionspartner gemeinsam und vollständig vor, besteht die Gefahr einer Spontanreaktion, bei der die exotherme Reaktionswärme nicht mehr genügend schnell abgeführt werden kann. Bei Benutzung von Alkoholen als Reaktionsmedium besteht weiterhin die Gefahr der unerwünschten Etherbildung mit dem Reaktionspartner Alkylhalogenid.

Es wurde ein Verfahren zur Herstellung von Nitrophenyl-alkylethern der Formel 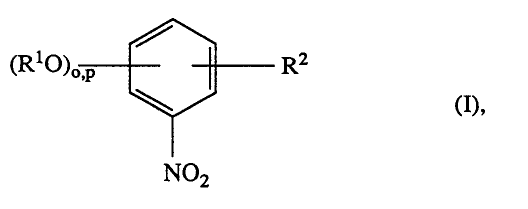
in der
- R¹: geradkettiges oder verzweigtes C₁-C₄-Alkyl bedeutet,
- o,p: die ortho- oder para-Position von R¹O zur Nitrogruppe anzeigt und
- R²: für Wasserstoff, geradkettiges oder verzweigtes C₁-C₈-Alkyl, C₃-C₈-Cycloalkyl, C₇-C₁₀-Aralkyl, C₆-C₁₂-Aryl, geradkettiges oder verzweigtes C₁-C₈-Alkoxy, geradkettiges oder verzweigtes C₁-C₈-Alkylthio, C₆-C₁₂-Aryloxy, C₆-C₁₂-Arylthio, -SO₂-R³, -SO₂-OR³, -SO₂NR³₂, -SOR³, Fluor, Chlor, oder Brom steht, wobei
R³ für C₁-C₄-Alkyl steht,
durch Umsetzung der zugrunde liegenden Nitrophenole mit Alkylchloriden in Gegenwart von Chlorwasserstoff bindenden Verbindungen gefunden, das dadurch gekennzeichnet ist, daß man ein Nitrophenol der Formel 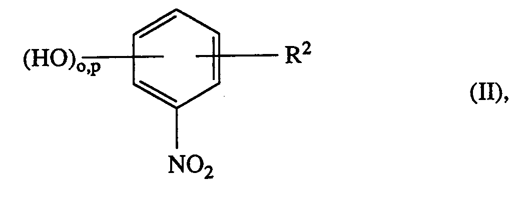
in der
- o,p: die ortho- oder para-Position der Hydroxylgruppe zur Nitrogruppe anzeigt und
- R²: die genannte Bedeutung hat,
mit 100-200 Mol-%, bezogen auf die Menge des Nitrophenols, eines Alkylchlorids der Formel

R¹-Cl (III),

in der
- R¹: die genannte Bedeutung hat,
in Gegenwart von 80-130 Äquivalent-%, bezogen auf die Menge des Alkylchlorids, einer Chlorwasserstoff bindenden Verbindung aus der Gruppe der Hydroxide, Carbonate und Hydrogencarbonate der (Erd)Alkalimetalle so umsetzt, daß das Nitrophenol in Wasser als Reaktionsmedium vorgelegt wird, wobei die Menge an Wasser mindestens ausreichen muß, um alle entstehenden anorganischen Salze zu lösen, und das Alkylchlorid und die Chlorwasserstoff bindende Verbindung simultan zugesetzt werden, wobei im Temperaturbereich von 20 bis 200°C und im Druckbereich von 1 bis 50 bar und in Abwesenheit oder in Gegenwart eines Bromids oder Iodids eines (Erd)Alkalimetalls in einer Menge von 0 bis 10 Mol-%, bezogen auf das umzusetzende Nitrophenol, gearbeitet wird.

Geradkettiges oder verzweigtes C₁-C₈-Alkyl ist beispielsweise Methyl, Ethyl, Propyl, Isopropyl, Butyl, Isobutyl, die isomeren Hexyle oder Octyle. Bevorzugtes Alkyl hat 1 bis 4 C-Atome und ist besonders bevorzugt Methyl oder Ethyl und ganz besonders bevorzugt Methyl.

C₃-C₈-Cycloalkyl ist beispielsweise Cyclopropyl, Cyclobutyl, Cyclopentyl, Cyclohexyl, Cycloheptyl oder Cyclooctyl, bevorzugt Cyclopropyl, Cyclopentyl oder Cyclohexyl.

C₇-C₁₀-Aralkyl ist beispielsweise Benzyl, Phenylethyl oder Phenylpropyl, bevorzugt Benzyl.

C₆-C₁₂-Aryl ist beispielsweise Phenyl, Naphthyl oder Biphenylyl, bevorzugt Phenyl.

Die Alkyl- bzw. Arylanteile in den weiteren Bedeutungen für R², nämlich Alkoxy, Alkylthio, Aryloxy oder Arylthio sind analog aufgebaut wie oben bereits beschrieben.

Nitrophenole der beschriebenen Art zum Einsatz im erfindungsgemäßen Verfahren und Methoden zu deren Herstellung sind dem Fachmann bekannt.

In bevorzugter Weise wird ein Nitrophenol der Formel 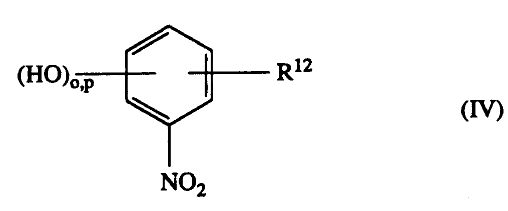
eingesetzt, worin
- o,p: die ortho- oder para-Position der Hydroxylgruppe zur Nitrogruppe anzeigt und
- R¹²: Wasserstoff, geradkettiges oder verzweigtes C₁-C₄-Alkyl, Benzyl, Phenyl, geradkettiges oder verzweigtes C₁-C₄-Alkoxy oder Phenoxy bedeutet.

In besonders bevorzugter Weise wird ein Nitrophenol der Formel 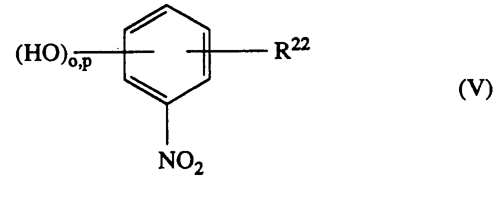
eingesetzt, worin
- o,p: die genannte Bedeutung hat und
- R²²: Wasserstoff oder geradkettiges oder verzweigtes C₁-C₄-Alkyl bedeutet.

In ganz besonders bevorzugter Weise wird ein Nitrophenol der Formel 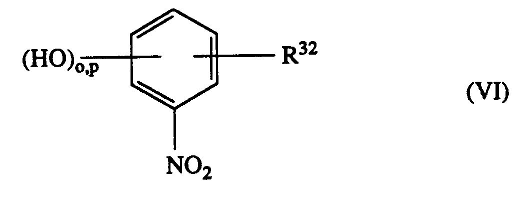
eingesetzt, worin
- o,p: die genannte Bedeutung hat und
- R³²: für Wasserstoff oder Methyl steht.

In den obigen Formeln für erfindungsgemäß einzusetzende Nitrophenole zeigen die Indices "o,p" die ortho- oder die para-Position der Hydroxylgruppe zur Nitrogruppe an, so daß erfindungsgemäß ortho-Nitrophenol oder para-Nitrophenol bzw. die davon abgeleiteten, durch R², R¹², R²² oder R³² substituierten Species eingesetzt werden.

In bevorzugter Weise werden jedoch die ortho-Nitrophenole eingesetzt.

In bevorzugter Weise wird daher erfindungsgemäß ein ortho-Nitrophenol der Formel 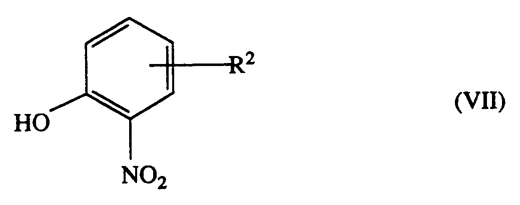
eingesetzt, worin
- R²: den oben beschriebenen Bedeutungsumfang hat.

In besonders bevorzugter Weise wird ein ortho-Nitrophenol der Formel 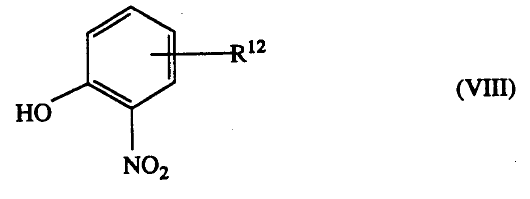
eingesetzt, worin
- R¹²: den beschriebenen Bedeutungsumfang hat.

In ganz besonders bevorzugter Weise wird ein ortho-Nitrophenol der Formel 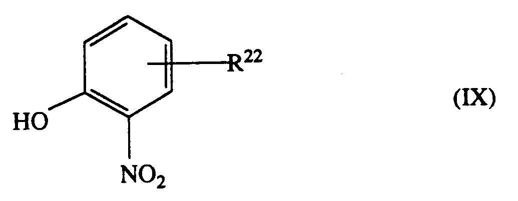
eingesetzt, worin
- R²²: die angegebene Bedeutung hat.

Ganz besonders wichtig ist das erfindungsgemäße Verfahren für den Einsatz eines ortho-Nitrophenols der Formel 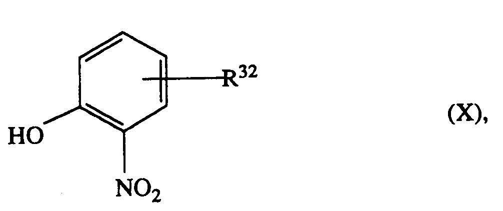
worin
- R³²: Wasserstoff oder Methyl bedeutet.

Die erfindungsgemäße Umsetzung wird mit einem Alkylchlorid der Formel (III) durchgeführt. Beispiele solcher Alkylchloride sind Methylchlorid, Ethylchlorid, Propylchlorid, Isopropylchlorid, 1-Butylchlorid, 2-Butylchlorid und Isobutylchlorid.

In bevorzugter Weise wird die Umsetzung mit einem Alkylchlorid der Formel

R¹¹-Cl (XI)

durchgeführt, worin
- R¹¹: Methyl oder Ethyl bedeutet.

In weiterhin bevorzugter Weise wird als Alkylhalogenid Methylchlorid eingesetzt.

Als Chlorwasserstoff bindende Verbindungen kommen die dem Fachmann bekannten Hydroxide, Carbonate und Hydrogencarbonate der (Erd)Alkalimetalle in Frage. In bevorzugter Weise sind die Chlorwasserstoff bindenden Verbindungen Hydroxide und/oder Carbonate von Natrium und/oder Kalium. In besonders bevorzugter Weise wird ein Gemisch von Natriumhydroxid und Kaliumhydroxid im Molverhältnis 5:1 bis 1:5 eingesetzt. Eine wegen der guten Dosiermöglichkeit verfahrenstechnisch besonders wichtige Variante ist der Einsatz eines solchen Gemisches von Natriumhydroxid und Kaliumhydroxid in Form einer 10 bis 60 gew.-%igen wäßrigen Lösung.

Das Reaktionsmedium im erfindungsgemäßen Verfahren ist Wasser. Es wird also auf organische Lösungsmittel, wie solche der vorne genannten Art, völlig verzichtet. Die Wassermenge wird so gewählt, daß alle entstehenden anorganischen Salze gelöst werden. Diese Wassermenge beträgt pro Mol des umzusetzenden Nitrophenols mindestens 150 g und umfaßt den Bereich 150 bis 1000 g, bevorzugt 150 bis 500 g pro Mol Nitrophenol.

Das erfindungsgemäße Verfahren kann in Abwesenheit oder in Gegenwart eines Bromids oder Iodids eines (Erd)Alkalimetalls durchgeführt werden. Für den Fall der Gegenwart eines solchen Salzes wird bevorzugt ein Bromid oder Iodid eines Alkalimetalls gewählt. Solche Salze sind nur in katalytischen Mengen erforderlich und umfassen die Menge 0 bis 10 Mol-%, bevorzugt 0 bis 5 Mol-%, besonders bevorzugt 0,1 bis 2 Mol-%, bezogen auf das umzusetzende Nitrophenol. Die Untergrenze von Null zeigt an, daß in einer großen Anzahl von Fällen auf einen solchen katalytischen Zusatz völlig verzichtet werden kann.

Ein solcher Verzicht ist beispielsweise möglich bei Verwendung von Kaliumhydroxid als Chlorwasserstoff bindender Verbindung; bei Verbindungen anderer Kationen werden vorteilhaft die genannten Bromide und Iodide zur Reaktionsbeschleunigung mitverwendet.

Es ist weiterhin erstaunlich, daß in einer solchen mehrphasigen Umsetzung auf Phasentransferkatalysatoren, wie beispielsweise quarternäre Ammoniumsalze oder Kronenether, völlig verzichtet werden kann.

Das erfindungsgemäße Verfahren wird im Druckbereich von 1 bis 50 bar, bevorzugt 2 bis 30 bar, besonders bevorzugt 2 bis 15 bar, und im Temperaturbereich von 20 bis 200°C, bevorzugt 50 bis 150°C, besonders bevorzugt 80 bis 130°C, durchgeführt.

Es ist ein weiteres Kennzeichen des erfindungsgemäßen Verfahrens, daß das Nitrophenol in Wasser als dem Reaktionsmedium vorgelegt wird und das Alkylchlorid und die Chlorwasserstoff bindende Substanz simultan zugesetzt werden. Die weiter oben genannte Gesamtmenge an Wasser setzt sich hierbei zusammen aus dem mit dem Nitrophenol gemeinsam vorgelegten Wasser und dem Wasser, welches zum Auflösen der Chlorwasserstoff bindenden Verbindung benötigt wird. Grundsätzlich ist es jedoch möglich, die Chlorwasserstoff bindende Verbindung als Feststoff gemeinsam mit dem Alkylchlorid zuzudosieren, wobei in einem solchen Fall die Gesamtmenge an Wasser gemeinsam mit dem Nitrophenol vorgelegt wird.

Die Menge an Alkylchlorid und der simultan zuzusetzenden, Chlorwasserstoff bindenden Verbindung beträgt im allgemeinen mindestens 100 Mol-% bzw. 100 Äquivalent-% des umzusetzenden Nitrophenols. Bei Benutzung geringerer Mengen an Alkylchlorid und Chlorwasserstoff bindender Verbindung ist die erfindungsgemäße Umsetzung grundsätzlich möglich, kann aber naturgemäß keine hohe Ausbeute ergeben. Zur Erzielung einer möglichst hohen Ausbeute werden hingegen Alkylchlorid und simultan dazu einzudosierende Chlorwasserstoff bindende Verbindung bevorzugt im Überschuß eingesetzt. Es wird also im Bereich von 100 bis 200 Mol-%, bevorzugt 120 bis 160 Mol-%, Alkylchlorid, bezogen auf das umzusetzende Nitrophenol und im Bereich von 80 bis 130 Äquivalent-%, bevorzugt 100 bis 110 Äquivalent-%, Chlorwasserstoff bindender Verbindung, bezogen auf das eingesetzte Alkylchlorid, gearbeitet.

Für den bevorzugten Fall der Verwendung von überschüssigem Alkylchlorid ist es weiterhin vorteilhaft, nach Zugabe etwa der stöchiometrischen Menge des Alkyl-chlorids (etwa 100 Mol-%) den darüber hinausgehenden Überschuß mit verringerter Geschwindigkeit gegenüber der Dosierung der stöchiometrischen Menge zuzusetzen. Bei der Zugabe des überschüssigen Alkylchlorids kann auch insoweit von der simultanen Zugabe der Chlorwasserstoff bindenden Verbindung abgewichen werden, als das Ende der Zugabe der Chlorwasserstoff bindenden Verbindung später liegt als das Ende der Zugabe des überschüssigen Alkylchlorids.

Wahrend der Durchführung des erfindungsgemäßen Verfahrens wird in einem geeigneten Rührgefäß, beispielsweise in einem Autoklav mit Rührwerk, gearbeitet. Nach Beendigung der Reaktion des erfindungsgemäßen Verfahrens wird das Reaktionsgemisch so aufgearbeitet, daß die organische und die wäßrige Phase getrennt werden, anschließend die wäßrige Phase mit einem geeigneten Lösungsmittel, beispielsweise mit Xylol, Toluol usw. extrahiert wird, die zuvor abgetrennte organische Phase mit dem Extrakt vereinigt wird und diese vereinigten organischen Phasen nach einer Wasserwäsche durch übliche Methoden, wie Destillation oder Kristallisation, zur Gewinnung der gewünschten Nitrophenyl-alkylether aufgearbeitet werden.

Das erfindungsgemäße Verfahren zeichnet sich durch eine Reihe von Vorteilen gegenüber Verfahren des Standes der Technik aus. So wird durch die Simultandosierung von Alkylierungsmittel und Chlorwasserstoff bindender Verbindung eine hohe Sicherheit gewonnen, da die stationäre Konzentration an Nitrophenolat sehr gering gehalten wird. Nitrophenolate neigen bekanntlich zu stark exothermer Zersetzung. Das erfindungsgemäße Verfahren vermeidet das Vorliegen von Nitrophenolat als einer weiteren festen Phase, wodurch die Alkylierung erleichtert wird und Anbackungen an den Reaktorwänden vermieden werden. Die Verwendung von Wasser als dem alleinigen Reaktionsmedium bringt sicherheitstechnische Vorteile gegenüber den bisher verwendeten brennbaren organischen Lösungsmitteln. Die Verwendung von Wasser als Reaktionsmedium bringt einen weiteren Vorteil mit sich, indem die bei der Aufarbeitung eines Reaktionsansatzes anfallenden Waschwässer in die Reaktion eines späteren Ansatzes zurückgeführt werden können. Weiterhin wird die Bildung von Ethern, die bei der Verwendung von Alkoholen als Lösungsmitteln auftreten, verhindert, wodurch die Abluftreinigung erleichert wird. Die erfindungsgemäße Vermeidung von Phasentransferkatalysatoren vermeidet die sonst stets gegebene Abwasserbelastung. Weiterhin ist es nicht erforderlich, das umzusetzende Nitrophenol in einem separaten Verfahrensschritt zuvor in das Phenolat umzuwandeln.

### Beispiel 1

o-Nitro-p-cresol wurde mit Methylchlorid in einem 7 l-Autoklav umgesetzt. Der Autoklav war mit einem Rührer, mit Dosiereinrichtungen sowie mit Temperatur- und Druckmessung ausgerüstet.

1610 g (10,52 Mol) o-Nitro-p-cresol wurden in 2630 g Wasser vorgelegt. Danach wurde auf 110°C aufgeheizt, wobei sich ein Eigendruck von 1,5 bar einstellte.

In 4 Stunden wurden unter Rühren simultan 991 g einer 50 %igen Alkalilauge (Molverhältnis NaOH:KOH = 7:3) und 532 g Chlormethan eindosiert, wobei der Druck bis auf 8 bar stieg. Danach wurden in 4 Stunden 566 g der Alkalilauge und, zur gleichen Zeit beginnend, jedoch bereits nach 2 Stunden beendet, 319 g Chlormethan eindosiert, wobei der Druck bis auf 10 bar stieg. Nach einer halben Stunde Nachreaktion wurde auf Raumtemperatur abgekühlt, der Autoklav entspannt und das Reaktionsgemisch entnommen. Hierzu wurden weitere 660 g Wasser zum Spülen benutzt.

Zur Aufarbeitung wurde der Autoklaveninhalt gemeinsam mit dem Spülwasser bis zu einer Sumpftemperatur von 105°C zur Entfernung aller flüchtigen Bestandteile erhitzt. Nach Abkühlung auf 55°C ließ man zur Trennung der organischen und der wäßrigen Phase absitzen. Die organische Phase wurde mit Wasser gewaschen, welches in einem folgenden Reaktionsansatz Verwendung finden kann. Die wäßrige Phase wurde mit Xylol extrahiert. Die gewaschene organische Phase und der Xylol-Extrakt wurden nochmals mit Wasser gewaschen und sodann am Rotationsverdampfer zur Entfernung des restlichen Wassers destilliert. Nach vollständiger destillativer Entfernung des Xylols erhielt man 1712 g 2-Nitro-4-methyl-methoxy-benzol in einer Reinheit von 98,4 %; das entspricht 1683,8 g einer 100 %igen Ware und somit 95,8 % der theoretischen Ausbeute.

### Beispiel 2

Es wurde wie in Beispiel 1 gearbeitet, jedoch wurden in den ersten 4 Stunden 758 g 50 %ige NaOH und in der zweiten Phase 708 g 50 %ige KOH eindosiert.
Nach Aufarbeitung wurde 2-Nitro-4-methyl-methoxybenzol in einer Ausbeute von 94,8 % der Theorie erhalten.

### Beispiel 3

Es wurde wie in Beispiel 1 gearbeitet, jedoch wurden in den ersten 4 Stunden 842 g 50 %ige NaOH und in der zweiten Phase 421 g 50 %ige NaOH und 266 g Chlormethan eindosiert.
Nach Aufarbeitung wurde 2-Nitro-4-methyl-methoxybenzol in einer Ausbeute von 92,3 % der Theorie erhalten.

### Beispiel 4

Es wurde wie in Beispiel 1 gearbeitet, jedoch wurden nur 2200ml Wasser vorgelegt. Ausbeute: 91,8 % der Theorie.

### Beispiel 5

Es wurde wie in Beispiel 1 gearbeitet, jedoch wurden 30 g Natriumiodid zusammen mit dem Wasser vorgelegt. In den ersten 4 Stunden wurden 842 g 50 %ige NaOH und in der zweiten Phase in 4 Stunden 505 g 50 %ige NaOH und in 2 Stunden - zur gleichen Zeit beginnend - 319 g Chlormethan eindosiert.
Ausbeute nach Aufarbeitung: 96,3 % der Theorie.

### Beispiel 6

Es wurde wie in Beispiel 1 gearbeitet, jedoch wurden 1610 g (10,52 Mol) 4-Nitro-3-methyl-phenol eingesetzt.
Nach Aufarbeitung wurden 1652 g 4-Nitro-3-methyl-anisol in einer Reinheit von 99 % isoliert; das entspricht einer Ausbeute von 93 % der Theorie. Erstarrungspunkt 46°C.

### Beispiel 7

Es wurde wie in Beispiel 1 gearbeitet, jedoch wurden 1465 g (10,52 Mol) o-Nitrophenol in 2630g Wasser bei 50°C vorgelegt.

In 4 Stunden wurden unter Rühren simultan 991 g einer 50 %igen Alkalilauge (Molverhältnis NaOH:KOH = 7:3) und 676 g Ethylchlorid eindosiert, wobei der Autoklav unter Eigendruck gehalten wurde.

Danach wurden in 4 Stunden 472 g Alkalilauge und, zur gleichen Zeit beginnend, 340 g Ethylchlorid in 2 Stunden eindosiert.
Nach Aufarbeitung wurden 1688 g o-Nitrophenetol in einer Reinheit von 99 % erhalten; das entspricht einer Ausbeute von 95 % der Theorie. Siedepunkt 144°C/10 mbar.

## Patentansprüche

1. Verfahren zur Herstellung von Nitrophenyl-alkylethern der Formel 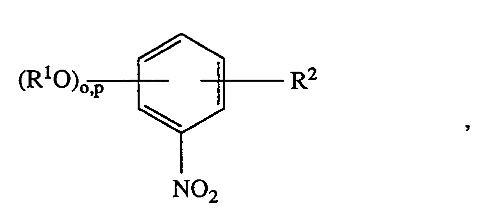 in der
R¹ geradkettiges oder verzweigtes C₁-C₄-Alkyl bedeutet,
o,p die ortho- oder para-Position von R¹O zur Nitrogruppe anzeigt und
R² für Wasserstoff, geradkettiges oder verzweigtes C₁-C₈-Alkyl, C₃-C₈-Cycloalkyl, C₇-C₁₀-Aralkyl, C₆-C₁₂-Aryl, geradkettiges oder verzweigtes C₁-C₈-Alkoxy, geradkettiges oder verzweigtes C₁-C₈-Alkylthio, C₆-C₁₂-Aryloxy, C₆-C₁₂-Arylthio, -SO₂-R³, -SO₂-OR³, -SO₂-NR³₂, -SOR³, Fluor, Chlor, oder Brom steht, wobei
R³ für C₁-C₄-Alkyl steht,
durch Umsetzung der zugrunde liegenden Nitrophenole mit Alkylchloriden in Gegenwart von Chlorwasserstoff bindenden Verbindungen, dadurch gekennzeichnet, daß man ein Nitrophenol der Formel 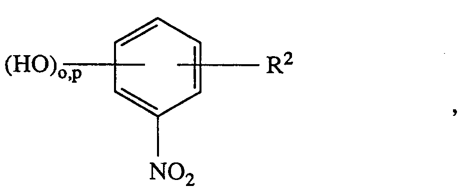 in der
o,p die ortho- oder para-Position der Hydroxylgruppe zur Nitrogruppe anzeigt und
R² die genannte Bedeutung hat,
mit 100-200 Mol-%, bevorzugt 120-160 Mol-%, bezogen auf die Menge des Nitrophenols, eines Alkylchlorids der Formel
R¹-Cl
,
in der
R¹ die genannte Bedeutung hat,
in Gegenwart von 80-130 Äquivalent-%, bevorzugt 100-110 Äquivalent-%, bezogen auf die Menge des Alkylchlorids, einer Chlorwasserstoff bindenden Verbindung aus der Gruppe der Hydroxide, Carbonate und Hydrogencarbonate der (Erd)Alkalimetalle so umsetzt, daß das Nitrophenol in Wasser als Reaktionsmedium vorgelegt wird, wobei die Menge an Wasser mindestens ausreichen muß, um alle entstehenden anorganischen Salze zu lösen, und das Alkylchlorid und die Chlorwasserstoff bindende Verbindung simultan zugesetzt werden, wobei im Temperaturbereich von 20 bis 200°C und im Druckbereich von 1 bis 50 bar und in Abwesenheit oder in Gegenwart eines Bromids oder Iodids eines (Erd)Alkalimetalls in einer Menge von 0 bis 10 Mol-%, bezogen auf das umzusetzende Nitrophenol, gearbeitet wird.

2. Verfahren nach Anspruch 1, dadurch gekennzeichnet, daß ein Nitrophenol der Formel 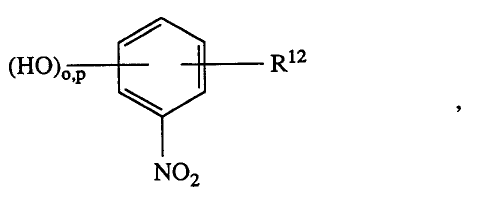 worin
o,p die ortho- oder para-Position der Hydroxylgruppe zur Nitrogruppe anzeigt und
R¹² Wasserstoff, geradkettiges oder verzweigtes C₁-C₄-Alkyl, Benzyl, Phenyl, geradkettiges oder verzweigtes C₁-C₄-Alkoxy oder Phenoxy bedeutet,
bevorzugt ein Nitrophenol der Formel 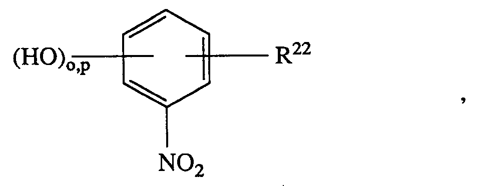 worin
o,p die genannte Bedeutung hat und
R²² Wasserstoff oder geradkettiges oder verzweigtes C₁-C₄-Alkyl bedeutet,
besonders bevorzugt ein Nitrophenol der Formel 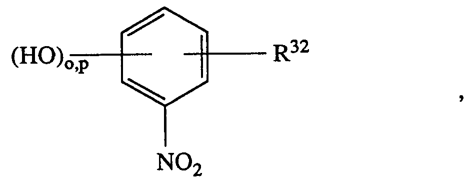 worin
o,p die genannte Bedeutung hat und
R³² für Wasserstoff oder Methyl steht,
eingesetzt wird.

3. Verfahren nach Anspruch 1, dadurch gekennzeichnet, daß ein ortho-Nitrophenol der Formel 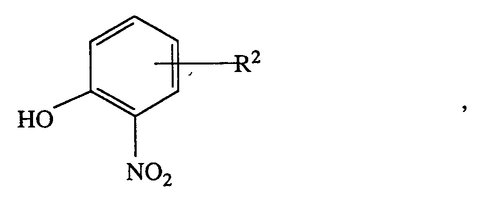 worin
R² für Wasserstoff, geradkettiges oder verzweigtes C₁-C₈-Alkyl, C₃-C₈-Cycloalkyl, C₇-C₁₀-Aralkyl, C₆-C₁₂-Aryl, geradkettiges oder verzweigtes C₁-C₈-Alkoxy, geradkettiges oder verzweigtes C₁-C₈-Alkylthio, C₆-C₁₂-Aryloxy, C₆-C₁₂-Arylthio, -SO₂-R³, -SO₂-OR³, -SO₂-NR³₂, -SOR³, Fluor, Chlor, oder Brom steht, wobei
R³ für C₁-C₄-Alkyl steht,
bevorzugt ein ortho-Nitrophenol der Formel 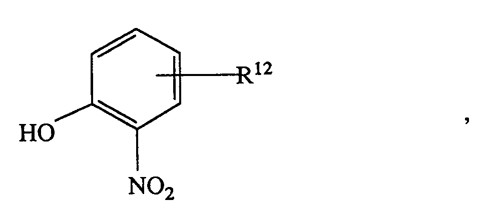 worin
R¹² Wasserstoff, geradkettiges oder verzweigtes C₁-C₄-Alkyl, Benzyl, Phenyl, geradkettiges oder verzweigtes C₁-C₄-Alkoxy oder Phenoxy bedeutet,
besonders bevorzugt ein ortho-Nitrophenol der Formel 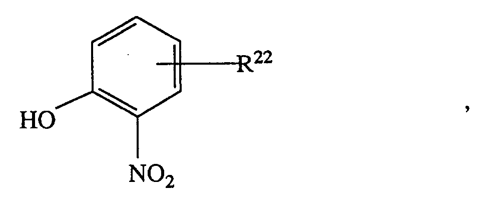 worin
R²² Wasserstoff oder geradkettiges oder verzweigtes C₁-C₄-Alkyl bedeutet,
ganz besonders bevorzugt ein ortho-Nitrophenol der Formel 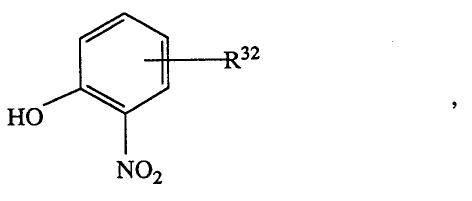 worin
R³² Wasserstoff oder Methyl bedeutet,
eingesetzt wird.

4. Verfahren nach Anspruch 1, dadurch gekennzeichnet, daß ein Alkylchlorid der Formel
R¹¹-Cl
,
worin
R¹¹ Methyl oder Ethyl bedeutet,
bevorzugt Methylchlorid, eingesetzt wird.

5. Verfahren nach Anspruch 1, dadurch gekennzeichnet, daß als Chlorwasserstoff bindende Verbindungen Hydroxide und/oder Carbonate von Natrium und/oder Kalium, bevorzugt ein Gemisch von NaOH und KOH im Molverhältnis 5:1 bis 1:5, besonders bevorzugt in Form einer 10 bis 60 gew.-%igen wäßrigen Lösung, eingesetzt werden.

6. Verfahren nach Anspruch 1, dadurch gekennzeichnet, daß die Wassermenge im Reaktionsansatz 150 bis 1000 g, bevorzugt 150 bis 500 g pro Mol umzusetzendes Nitrophenol, beträgt.

7. Verfahren nach Anspruch 1, dadurch gekennzeichnet, daß in Abwesenheit oder in Gegenwart eines Bromids oder Iodids eines Alkalimetalls in einer Menge von 0 bis 5 Mol-%, bevorzugt von 0,1 bis 2 Mol-%, bezogen auf das umzusetzende Nitrophenol, gearbeitet wird.

8. Verfahren nach Anspruch 1, dadurch gekennzeichnet, daß im Druckbereich von 2 bis 30 bar, bevorzugt 2 bis 15 bar, gearbeitet wird.

9. Verfahren nach Anspruch 1, dadurch gekennzeichnet, daß im Temperaturbereich von 50 bis 150°C, bevorzugt 80 bis 130°C, gearbeitet wird.

10. Verfahren nach Anspruch 1, dadurch gekennzeichnet, daß bei Verwendung überschüssiger Mengen an Alkylchlorid, bezogen auf Nitrophenol, die über die stöchiometrische Menge hinausgehende Menge an Alkylchlorid mit verringerter Geschwindigkeit gegenüber der Dosierung der stöchiometrischen Menge zugesetzt wird und hierbei das Ende der Zugabe der Chlorwasserstoff bindenden Verbindung später liegt als das Ende der Zugabe des Alkylchlorids.

## Claims

1. Process for the preparation of nitrophenyl alkyl ethers of the formula 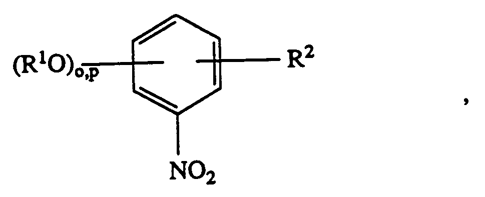 in which
R¹ is straight-chain or branched C₁-C₄-alkyl,
o,p indicates the ortho- or para-position of R¹O to the nitro group and
R² represents hydrogen, straight-chain or branched C₁-C₈-alkyl, C₃-C₈-cycloalkyl, C₇-C₁₀-aralkyl, C₆-C₁₂-aryl, straight-chain or branched C₁-C₈-alkoxy, straight-chain or branched C₁-C₈-alkylthio, C₆-C₁₂-aryloxy, C₆-C₁₂-arylthio, -SO₂-R³, -SO₂-OR³, -SO₂-NR³₂, -SOR³, fluorine, chlorine or bromine, in which
R³ represents C₁-C₄-alkyl,
by reacting the base nitrophenols with alkyl chlorides in the presence of hydrogen chloride-binding compounds, characterised in that a nitrophenol of the formula 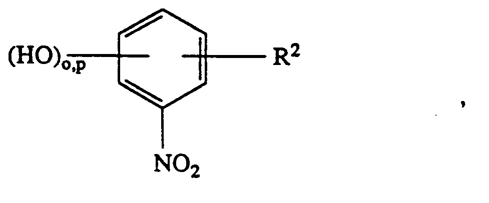 in which
o,p indicates the ortho- or para-position of the hydroxyl group to the nitro group and
R² has the meaning given above,
is reacted with 100-200 mol%, preferably 120-160 mol%, relative to the amount of the nitrophenol, of an alkyl chloride of the formula
R¹-Cl
,
in which
R¹ has the meaning given above,
in the presence of 80-130 equivalent%, preferably 100-110 equivalent%, relative to the amount of the alkyl chloride, of a hydrogen chloride-binding compound selected from the group consisting of the hydroxides, carbonates and hydrogen carbonates of the alkali(ne earth) metals in such a manner that the nitrophenol is introduced into the water used as reaction medium, where the quantity of water must at least be sufficient to dissolve all resulting inorganic salts, and the alkyl chloride and the hydrogen chloride-binding compound are added simultaneously, and where the reaction is carried out in the temperature range from 20 to 200°C and in the pressure range from 1 to 50 bar, and in the absence or presence of a bromide or iodide of an alkali(ne earth) metal in an amount of 0 to 10 mol%, relative to the nitrophenol to be reacted.

2. Process according to Claim 1, characterised in that a nitrophenol of the formula 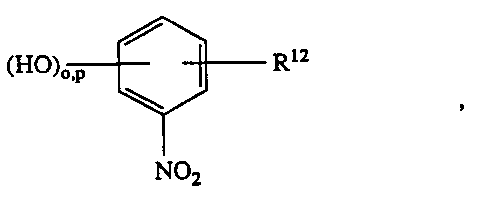 in which
o,p indicates the ortho- or para-position of the hydroxyl group to the nitro group and
R¹² is hydrogen, straight-chain or branched C₁-C₄-alkyl, benzyl, phenyl, straight-chain or branched C₁-C₄-alkoxy or phenoxy,
preferably a nitrophenol of the formula 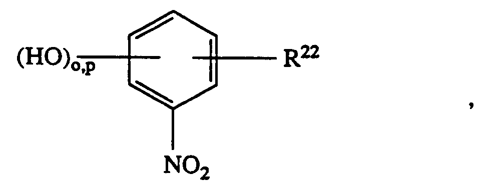 in which
o,p has the meaning given above and
R²² is hydrogen or straight-chain or branched C₁-C₄-alkyl,
particularly preferably a nitrophenol of the formula 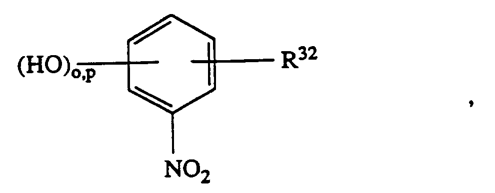 in which
o,p has the meaning given above and
R³² represents hydrogen or methyl,
is used.

3. Process according to Claim 1, characterised in that an ortho-nitrophenol of the formula 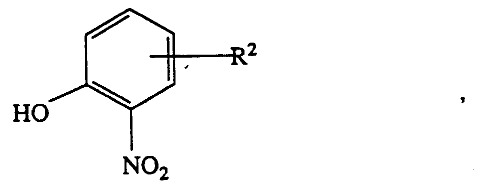 in which
R² represents hydrogen, straight-chain or branched C₁-C₈-alkyl, C₃-C₈-cycloalkyl, C₇-C₁₀-aralkyl, C₆-C₁₂-aryl, straight-chain or branched C₁-C₈-alkoxy, straight-chain or branched C₁-C₈-alkylthio, C₆-C₁₂-aryloxy, C₆-C₁₂-arylthio, -SO₂-R³, -SO₂-OR³, -SO₂-NR³₂, -SOR³, fluorine, chlorine or bromine, in which
R³ represents C₁-C₄-alkyl,
preferably an ortho-nitrophenol of the formula 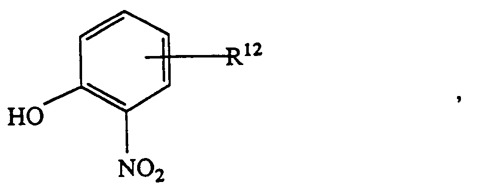 in which
R¹² is hydrogen, straight-chain or branched C₁-C₄-alkyl, benzyl, phenyl, straight-chain or branched C₁-C₄-alkoxy or phenoxy,
particularly preferably an ortho-nitrophenol of the formula 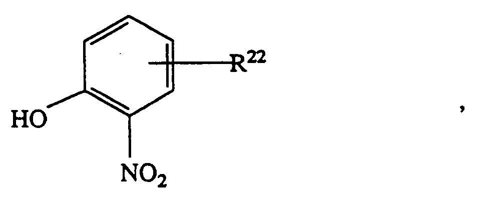 in which
R²² is hydrogen or straight-chain or branched C₁-C₄-alkyl,
very particularly preferably an ortho-nitrophenol of the formula 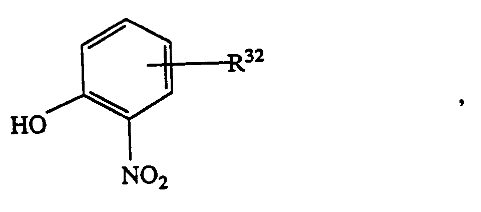 in which
R³² is hydrogen or methyl,
is used.

4. Process according to Claim 1, characterised in that an alkyl chloride of the formula
R¹¹-Cl
,
in which
R¹¹ is methyl or ethyl,
preferably methyl chloride, is used.

5. Process according to Claim 1, characterised in that the hydrogen chloride-binding compounds used are hydroxides and/or carbonates of sodium and/or potassium, preferably a mixture of NaOH and KOH in a molar ratio of 5:1 to 1:5, particularly preferably in the form of a 10 to 60% strength by weight aqueous solution.

6. Process according to Claim 1, characterised in that the quantity of water in the reaction mixture is 150 to 1000 g, preferably 150 to 500 g per mole of nitrophenol to be reacted.

7. Process according to Claim 1, characterised in that it is carried out in the absence or presence of a bromide or iodide of an alkali metal in a quantity of 0 to 5 mol%, preferably 0.1 to 2 mol%, relative to the nitrophenol to be reacted.

8. Process according to Claim 1, characterised in that it is carried out in the pressure range from 2 to 30 bar, preferably 2 to 15 bar.

9. Process according to Claim 1, characterised in that it is carried out in the temperature range from 50 to 150°C, preferably 80 to 130°C.

10. Process according to Claim 1, characterised in that when excess amounts of alkyl chloride, relative to nitrophenol, are used, the amount of alkyl chloride in excess of the stoichiometric amount is added at a reduced rate compared to the metering of the stoichiometric amount, and the completion of the addition of the hydrogen chloride-binding compound occurs later than the completion of the addition of the alkyl chloride.

## Revendications

1. Procédé pour la préparation d'éthers nitrophénylalkyliques répondant à la formule 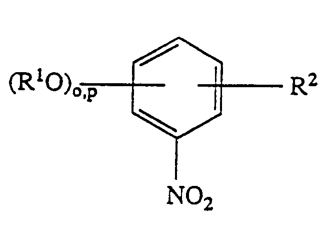 dans laquelle
R¹ représente un groupe alkyle en C₁-C₄ à chaîne droite ou ramifiée,
o,p indique la position ortho ou para de R¹O par rapport au groupe nitro, et
R² représente un atome d'hydrogène, un groupe alkyle en C₁-C₈ à chaîne droite ou ramifiée, un groupe cycloalkyle en C₃-C₈, un groupe aralkyle en C₇-C₁₀, un groupe aryle en C₆-C₁₂, un groupe alcoxy en C₁-C₈ à chaîne droite ou ramifiée, un groupe alkyl(en C₁-C₈)thio à chaîne droite ou ramifiée, un groupe aryl(en C₆-C₁₂)oxy, un groupe aryl(en C₆-C₁₂)thio, -SO₂-R³, -SO₂-OR³, -SO₂-NR³₂, -SOR³, un atome de fluor, un atome de chlore ou un atome de brome, où
R³ représente un groupe alkyle en C₁-C₄,
par la mise en réaction des nitrophénols de départ avec des chlorures d'alkyle en présence de composés neutralisant l'acide chlorhydrique, caractérisé en ce qu'on fait réagir un nitrophénol de formule 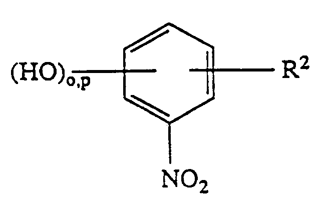 dans laquelle
o,p indique la position ortho ou para du groupe hydroxyle par rapport au groupe nitro, et
R² a la signification mentionnée,
avec 100-200 moles %, de préférence 120-160 moles %, rapportées à la quantité du nitrophénol, d'un chlorure d'alkyle de formule
R¹-Cl
dans laquelle
R¹ a la signification mentionnée,
en présence de 80-130 équivalents %, de préférence de 100-110 équivalents %, rapportés à la quantité du chlorure d'alkyle, d'un composé neutralisant l'acide chlorhydrique, choisi parmi le groupe des hydroxydes, des carbonates et des hydrogénocarbonates des métaux alcalins (alcalino-terreux), de telle sorte que l'on dépose au préalable le nitrophénol dans de l'eau comme milieu réactionnel, dans lequel la quantité d'eau doit au moins être suffisante pour dissoudre tous les sels inorganiques qui se forment, et on ajoute simultanément le chlorure d'alkyle et le composé neutralisant l'acide chlorhydrique en travaillant dans un domaine de température de 20 à 200°C et dans un domaine de pression de 1 à 50 bar et en l'absence ou en présence d'un bromure ou d'un iodure d'un métal alcalin (alcalino-terreux) en une quantité de 0 à 10 moles %, rapportée au nitrophénol qui doit être mis à réagir.

2. Procédé selon la revendication 1, caractérisé en ce qu'on met en oeuvre un nitrophénol répondant à la formule 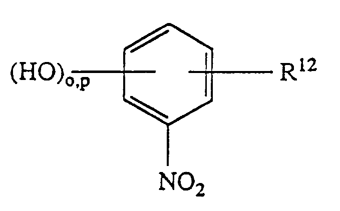 dans laquelle
o,p indique la position ortho ou para du groupe hydroxyle par rapport au groupe nitro et
R¹² représente un atome d'hydrogène, un groupe alkyle en C₁-C₄ à chaîne droite ou ramifiée, un groupe benzyle, un groupe phényle, un groupe alcoxy en C₁-C₄ à chaîne droite ou ramifiée ou un groupe phénoxy,
de préférence un nitrophénol répondant à la formule 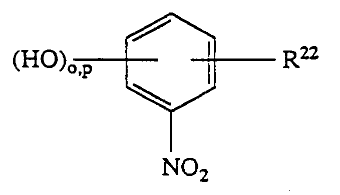 dans laquelle
o,p a la signification indiquée et
R²² représente un atome d'hydrogène ou un groupe alkyle en C₁-C₄ à chaîne droite ou ramifiée,
de manière particulièrement préférée, un nitrophénol répondant à la formule 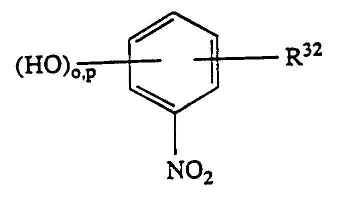 dans laquelle
o,p a la signification indiquée et
R³² représente un atome d'hydrogène ou un groupe méthyle.

3. Procédé selon la revendication 1, caractérisé en ce qu'on met en oeuvre un ortho-nitrophénol répondant à la formule 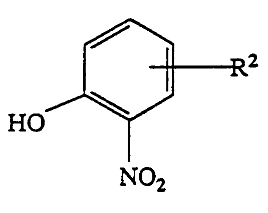 dans laquelle
R² représente un atome d'hydrogène, un groupe alkyle en C₁-C₈ à chaîne droite ou ramifiée, un groupe cycloalkyle en C₃-C₈, un groupe aralkyle en C₇-C₁₀, un groupe aryle en C₆-C₁₂, un groupe alcoxy en C₁-C₈ à chaîne droite ou ramifiée, un groupe alkyl(en C₁-C₈)thio à chaîne droite ou ramifiée, un groupe aryl(en C₆-C₁₂)oxy, un groupe aryl(en C₆-C₁₂)thio, -SO₂-R³, -SO₂-OR³, -SO₂-NR³₂, -SOR³, un atome de fluor, un atome de chlore ou un atome de brome, où
R³ représente un groupe alkyle en C₁-C₄,
de préférence un ortho-nitrophénol répondant à la formule 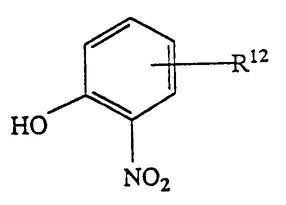 dans laquelle
R¹² représente un atome d'hydrogène, un groupe alkyle en C₁-C₄ à chaîne droite ou ramifiée, un groupe benzyle, un groupe phényle, un groupe alcoxy en C₁-C₄ à chaîne droite ou ramifiée ou un groupe phénoxy,
de manière particulièrement préférée, un ortho-nitrophénol répondant à la formule 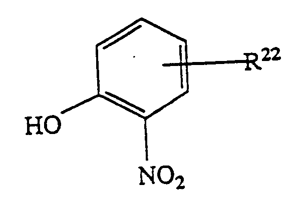 dans laquelle
R²² représente un atome d'hydrogène ou un groupe alkyle en C₁-C₄ à chaîne droite ou ramifiée,
de manière tout particulièrement préférée, un orthonitrophénol répondant à la formule 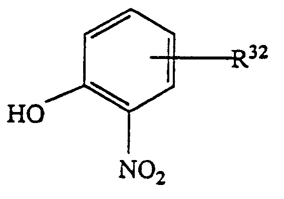 dans laquelle
R³² représente un atome d'hydrogène ou un groupe méthyle.

4. Procédé selon la revendication 1, caractérisé en ce qu'on met en oeuvre un chlorure d'alkyle répondant à la formule
R¹¹-Cl
dans laquelle
R¹¹ représente un groupe méthyle ou un groupe éthyle,
de préférence un chlorure de méthyle.

5. Procédé selon la revendication 1, caractérisé en ce que, comme composés neutralisant l'acide chlorhydrique, on met en oeuvre des hydroxydes et/ou des carbonates de sodium et/ou de potassium, de préférence un mélange de NaOH et de KOH dans le rapport molaire 5:1 à 1:5, de manière particulièrement préférée sous forme d'une solution aqueuse de 10 à 60% en poids.

6. Procédé selon la revendication 1, caractérisé en ce que la quantité d'eau, dans la charge réactionnelle, s'élève de 150 à 1.000 g, de préférence de 150 à 500 g par mole du nitrophénol qui doit être mis à réagir.

7. Procédé selon la revendication 1, caractérisé en ce qu'on travaille en l'absence ou en présence d'un bromure ou d'un iodure d'un métal alcalin en une quantité de 0 à 5 moles %,_{,} de préférence de 0,1 à 2 moles %, rapportée au nitrophénol qui doit être mis à réagir.

8. Procédé selon la revendication 1, caractérisé en ce qu'on travaille dans le domaine de pression de 2 à 30 bar, de préférence de 2 à 15 bar.

9. Procédé selon la revendication 1, caractérisé en ce qu'on travaille dans le domaine de température de 50 à 150°C, de préférence de 80 à 130°C.

10. Procédé selon la revendication 1, caractérisé en ce qu'en utilisant des quantités en excès de chlorure d'alkyle, rapportées au nitrophénol, on ajoute la quantité de chlorure d'alkyle qui dépasse la quantité stoechiométrique avec une vitesse réduite par rapport au dosage de la quantité stoechiométrique et, en l'occurrence, le terme de l'addition du composé neutralisant l'acide chlorhydrique est plus tardif que le terme de l'addition du chlorure d'alkyle.
